# EUROPÄISCHE PATENTSCHRIFT

(11) **EP 2 450 010 B1**
(45) Veröffentlichungstag und Bekanntmachung des Hinweises auf die Patenterteilung: **20.11.2013**
(21) Anmeldenummer: 11185355.2
(22) Anmeldetag: 17.10.2011
(51) Int. Cl.: A61F 2/958, A61M 25/10, A61F 2/966

(54) **Ballon-Katheter, insbesondere zum Ausbringen von Medikamenten oder Stents im Bereich einer Stenose**
Balloon catheter, in particular for delivering drugs or stents in the region of a stenosis
Cathéter à ballonnet, notamment destiné à l'administration de médicaments ou de stents dans la zone d'une sténose

(30) Priorität: 09.11.2010 US 411485 P
(43) Veröffentlichungstag der Anmeldung: 09.05.2012
(73) Patentinhaber: Biotronik AG, 8180 Bülach (CH)
(72) Erfinder: Wesselmann, Matthias, 8912 Glattfelden (CH); Brunner, Tobias, 8057 Zürich (CH); Lang, Hans, 8107 Buchs (CH)
(74) Vertreter: Lindner-Vogt, Karin L.

(56) Entgegenhaltungen:
- EP-A1- 0 292 587
- EP-A1- 1 836 998
- US-A1- 2005 080 474

## Beschreibung

Die Erfindung betrifft einen Ballon-Katheter, insbesondere zum Ausbringen von Medikamenten oder Stents im Bereich einer Stenose, welcher einen Außenschaft, einen Innenschaft und einen durch ein zuführbares Druckfluid dilatierbaren Ballon am distalen Katheterende aufweist. Der Ballon ist mit seinem proximalen Ende am Außenschaft und mit seinem distalen Ende am Innenschaft angebracht, der über den Außenschaft nach distal vorsteht.

Zum Hintergrund der Erfindung ist festzuhalten, dass für das Ausbringen von Stents im Bereich von Stenosen oder Läsionen in einem Körpergefäß oder für das Freisetzen von Medikamenten in solchen Körperregionen Katheter unterschiedlichster Bauart verwendet werden. So zeigt die US 2009/0018501 A1 einen Ballon-Katheter, dessen Ballon mit einem Medikament beschichtet ist. Der Ballon ist nach Art einer Rollmembran zwischen Außen- und Innenschaft angelegt. Beim Einführen des Katheters in den Körper ist der Innenschaft relativ zum Außenschaft nach proximal verschoben, sodass der Ballon zwischen Außen- und Innenschaft abgedeckt liegt. Am Einsatzort wird dann der Ballon mithilfe eines Druckfluids aufgeblasen, wobei der Außenschaft relativ zum Innenschaft nach proximal verschoben und der Ballon nach Art einer Rollmembran sich rollend und scherend öffnet und dilatiert. Problematisch ist hierbei die Tatsache, dass beim Öffnen des Rollmembran-Ballons dessen Membran sehr kleinen Biegeradien und hohen Scherkräften unterworfen ist, sodass die Beschichtung mit dem Wirkstoff vom Ballon unkontrolliert abgeschält werden kann. Auch können beim Öffnen des Ballons oder bereits auf dem Weg zum Einsatzort Wirkstoffverluste eintreten.

Im Zusammenhang mit dem Ausbringen von Stents zeigt die US 2009/0259286 A1 einen Katheter, bei dem der Stent während des Einführens des Katheters unter einer Hülle am distalen Ende in der kontrahierten Stellung gehalten ist. Diese Hülle ist so konzipiert, dass sie beim Dilatieren des Ballons und dadurch beim Aufspreizen des Stents entlang eines vorbestimmten Wegs aufreißt. Dies bedingt eine relativ komplizierte und fehleranfällige Auslegung der Hülle. Entsprechend ungenau kann die Positionierung des Stents sein.

Aus der US 2005/0033402 A1 ist schließlich eine Ausbringvorrichtung für einen Stent bekannt, bei dem Letzterer beim Zuführen unter einer Hülle abgedeckt angeordnet ist. Für das Freisetzen des Stents ist die Hülle mit einem Zugdraht versehen, über den von proximal her ein Wegziehen der Hülle und ein entsprechendes Freisetzen des Stents bewerkstelligt werden kann. Dies bedingt natürlich eine äußerst aufwändige Konstruktion des Katheters. Ferner ist der Zugdraht anfällig gegen ein Reißen. In diesem Falle ist ein Freisetzen des Stents nicht mehr möglich.

In der EP 0 596 145 B1 ist eine Methode beschrieben, mit der die Rückhaltekraft des Stents auf dem Katheter mittels Prägung des Stentmusters unter Temperatureinwirkung erreicht wird. Da der Ballon sich unter Wärmeeinwirkung verkürzt, ist die Einbettung mit dieser Methode nur dann optimal, wenn der Stent selber unter Wärmeinwirkung in den Ballon eingebettet und der Stent quasi im Ballonmaterial "eingefroren" wird. Bei Medikamenten-beschichteten Stents ist diese Methode der Stentfixierung nur anwendbar, solange die Glastemperatur des Ballonmaterials weit unterhalb der Zersetzungstemperatur des Medikaments liegt.

Aus der US 7,651,525 B2 ist eine Stent-Vorrichtung bekannt, bei der ein Katheter eine vom distalen zum proximalen Ende durchgehende äußere Hülse aufweist, in deren distalen Endbereich ein selbst-expandierender Stent auf einen dilatierbaren Ballon in kontrahiertem Zustand angeordnet ist. Zum Ausbringen des Stents wird die äußere Hülse in proximaler Richtung manuell durch einen Behandler oder durch eine mechanische Betätigung gegenüber dem Stent zurückgezogen. Dieser wird unter Aufblasen des Ballons und Selbstextrahierung an der Behandlungsstelle in einem Körpergefäß positioniert. EP 0 292 587 A1, US 2005/080474 A1 und EP 1 836 998 A1 offenbaren weitere Ballon-Katheter Systeme.

Ausgehend von den geschilderten Problemen des Standes der Technik liegt der Erfindung die Aufgabe zugrunde, einen Ballon-Katheter zum Ausbringen von Medikamenten oder Stents so zu verbessern, dass dies bei einfachem Handling und geringem konstruktiven Aufwand für den Katheter sicher und genau und im Falle einer Medikamentenfreisetzung wirkstoffschonend erfolgt.

Diese Aufgabe wird durch den Ballon-Katheter des Anspruchs 1 gelöst, wonach eine in einer Abdeckstellung über dem kontrahierten Ballon sitzende, auf dem Außenschaft verschiebbar geführte Abdeckhülse vorgesehen ist, die durch den dilatierenden Ballon unter dessen fortschreitender Freigabe von distal nach proximal über ihre Frontöffnung auf dem Außenschaft nach proximal verschiebbar ist.

Aufgrund dieser Ausgestaltung ist für das Freisetzen des Ballons durch das Rückziehen der Abdeckhülse kein gesonderter Mechanismus mehr notwendig, vielmehr wird die Abdeckhülse durch den dilatierenden Ballon selbsttätig verschoben, wodurch der Ballon immer weiter freigesetzt wird. Da dieser Freisetzvorgang vom distalen Ende des Ballons aus startet, kann der Ballon-Katheter sich jenseits einer Stenose im Gefäß verankern, sodass der Blutfluss im Gefäß dadurch gestoppt wird. Damit kann insbesondere ein selbstexpandierender Stent genau positioniert und ohne Springneigung beim Öffnen ausgebracht werden.

Medikamenten-beschichtete Stents können mit diesem Katheter sicher unterhalb der Abdeckhülse zur Stenose transportiert werden, ohne dass der Stent im Ballon gesondert eingebettet werden muss. Die thermische Belastung der Medikamentenbeschichtung, die zum Fixieren des Stents auf dem Ballon nötig ist, entfällt somit. Neigen Stentsegmente beim Durchfahren von engen Kurven zum Aufrichten ("fish scaling"), so sorgt die Abdeckhülse dafür, dass diese Segmente sich nicht im Körper oder am Führungskatheter verhaken und so den Stent abstreifen können.

Auch ist keine gesonderte Betätigung der Abdeckhülse notwendig, da diese quasi automatisch vom Ballon verschoben wird. Im Falle eines Ballons mit einer Medikamentenbeschichtung geht durch das Dilatieren des Ballons vom distalen Ende her und die dadurch verbundene Verankerung des Ballons im Gefäß mit entsprechendem Stocken des Blutstroms kein Wirkstoffverlust einher, da kein Blutstrom mehr vorhanden ist, der das Medikament wegschwemmen könnte. Beim Freisetzen des Ballons wird - anders als bei dem eingangs geschilderten Rollmembran-Ballon - die Beschichtung keinen engen Biegeradien und Scherbrückungen unterworfen.

Gemäß bevorzugten Ausführungsformen der Erfindung kann die axiale Reibkraft zwischen Ballon bzw. Stent und Hülle stark reduziert werden, indem im Bereich zwischen Ballon und Hülle ein Gegendruck zum Balloninnendruck erzeugt wird. Dazu ist vorzugsweise die Abdeckhülse druckdicht auf dem Außenschaft verschiebbar geführt.

Schließlich kann der Druckausgleich im Zwischenraum zwischen Abdeckhülse und Ballon mit dem zum Dilatieren des Ballons eingesetzten Druckfluid vorgenommen werden, indem beispielsweise der Zwischenraum zwischen Abdeckhülse und Ballon über eine Durchlassöffnung im Außenschaft mit der Druckfluidleitung zum Ballon kommuniziert.

Gemäß einer weiteren bevorzugten Ausführungsform der Erfindung kann der Ballon-Katheter verwendet werden, um selbst expandierende Stents zu setzen. Dazu wird der Stent auf dem Ballon durch die Abdeckhülse in ihrer Abdeckstellung im kontrahierten Zustand gehalten und durch das Dilatieren des Ballons allmählich freigesetzt.

Gemäß einer alternativen Ausführungsform dient die Abdeckhülse zum sicher abgedeckten Transport von wirkstoffbeschichteten Ballons zum Bereich einer Stenose und einer - wie oben bereits erwähnt - wirkstoffschonenden Freisetzung.

Gemäß einer weiteren bevorzugten Ausführungsform ist auf dem Außenschaft ein Anschlag zur Begrenzung des Verschiebewegs der Abdeckhülse nach proximal angeordnet. Damit kann die Beweglichkeit der Abdeckhülse so begrenzt werden, dass sie zwischen dem Konus des dilatierten Ballons und dem Anschlag eingeklemmt wird, sodass eine gute Abdichtung zwischen der Frontöffnung der Abdeckhülse und dem Ballonkonus stattfindet. Der Ballon kann damit mit dem maximalen Solldruck beaufschlagt werden, ohne dass die Gefahr besteht, dass das Druckfluid über den ebenfalls unter Druck gesetzten Zwischenraum zwischen Ballon und Abdeckhülse entweicht.

Das Abstreifen der Abdeckhülse nach distal kann konstruktiv durch einen Endanschlag verhindert werden, der größer ist als der proximale Abdeckhülsendurchmesser. Der Endanschlag kann als Ring aufgecrimpt, aufgeklebt oder durch Umformen des Außenschafts erzeugt werden.

Um eine zuverlässige allmähliche Öffnung des Ballons beim Dilatieren von distal nach proximal zu fördern, ist es von Vorteil, dass in der Abdeckstellung der Abdeckhülse zwischen der Frontöffnung und einem Konuskörper am distalen Ende des Innenschafts ein Initialöffnungsspalt für den Ballon verbleibt.

Das sichere Entleeren des Ballons beim Deflatieren kann durch zwei Maßnahmen sichergestellt werden: einerseits durch das Einstellen eines höheren Fließwiderstandes in Richtung Abdeckhülse z.B. durch eine nur sehr kleine Austrittsöffnung im Vergleich zum großen Fließquerschnitt in Richtung Ballon, oder andererseits durch das Ergänzen eines Rückschlagventils durch eine sehr dünne, nachgiebige, ringförmige Membran, die außen auf dem Außenschaft über dem Austrittsloch aufgeschrumpft wird. Beim Inflatieren hebt diese Membran ab und versorgt die Abdeckhülse mit dem nötigen Gegendruck. Beim Deflatieren mit maximal 1 bar Druckdifferenz verschließt diese Membran sicher das Austrittsloch. Diese beiden Maßnahmen können auch kombiniert werden.

Weitere Merkmale, Einzelheiten und Vorteile der Erfindung ergeben sich aus der nachfolgenden Beschreibung eines Ausführungsbeispiels anhand der beigefügten Zeichnungen. Es zeigen:

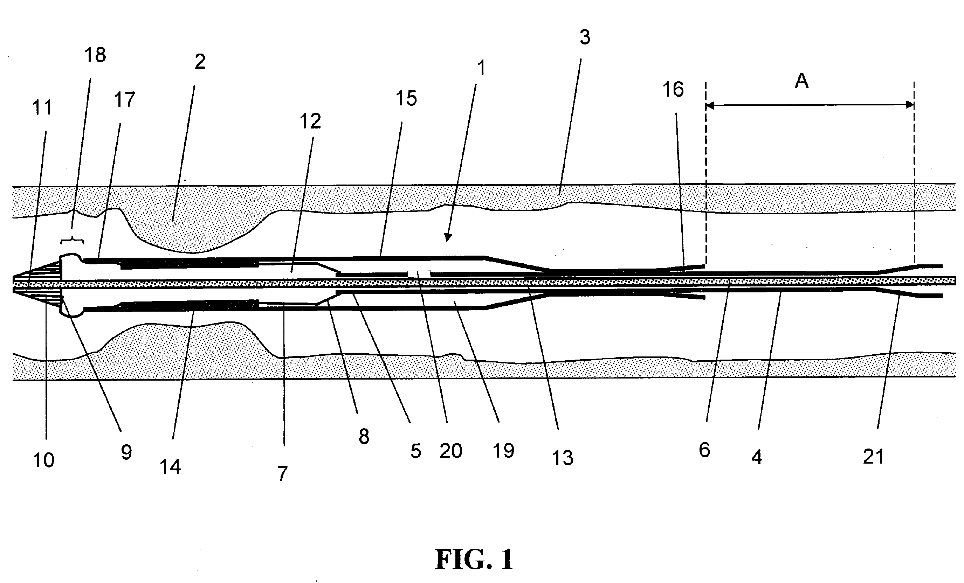

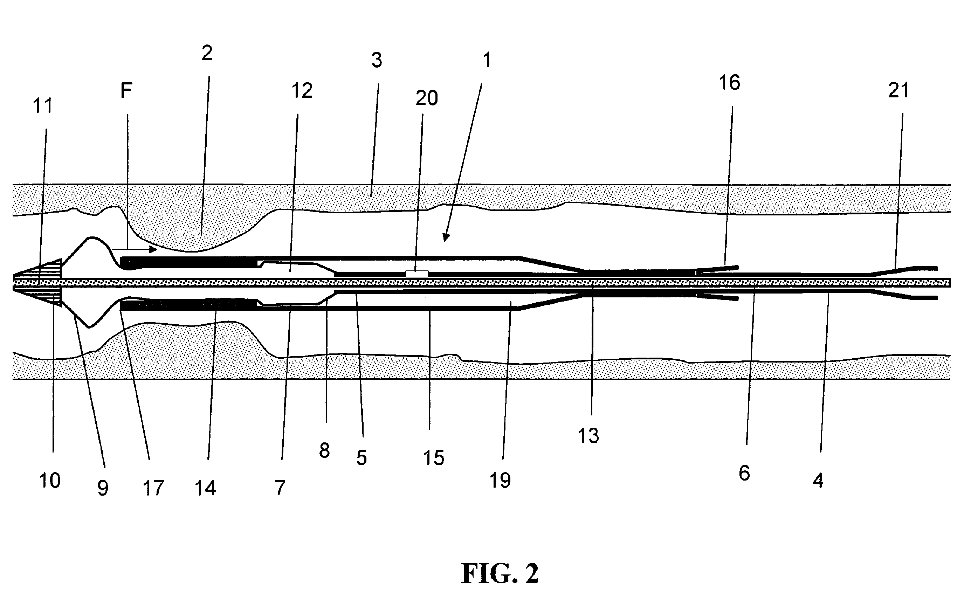

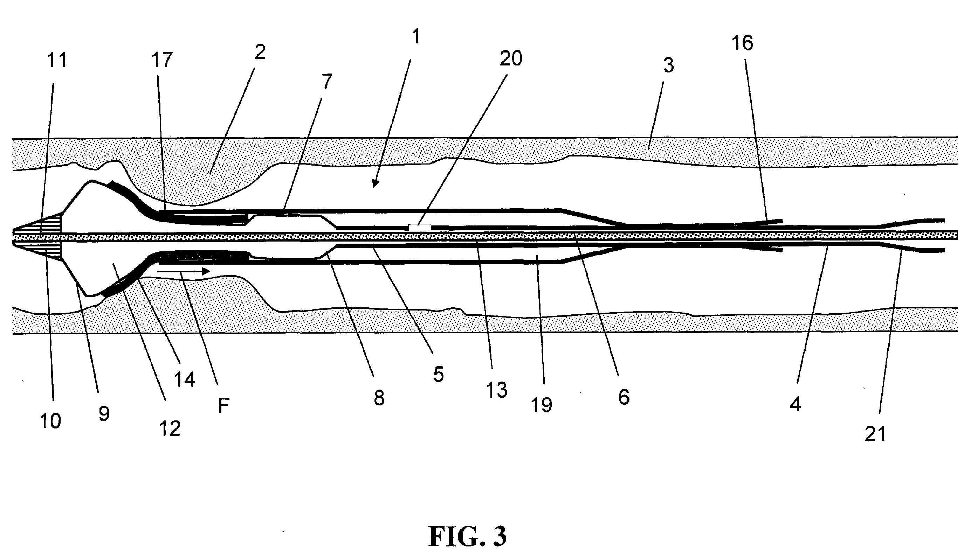

- Fig. 1 bis 8: schematische Längsaxialschnitte des distalen Endes eines Ballon-Katheters im Bereich einer Gefäß-Stenose in aufeinander folgenden Dila-tationsschritten des Ballons.

Die Figuren zeigen den distalen Endbereich eines Ballon-Katheters 1, der im Bereich einer Stenose 2 in einem Körpergefäß 3 platziert ist. Der Ballon-Katheter 1 weist einen lang gestreckten, flexiblen Außenschaft 4 und einen darin verlaufenden, sich über das distale Ende 5 des Außenschafts 4 hinaus erstreckenden Innenschaft 6 ebenfalls aus einem biegsamen Material auf. Ein durch ein Druckfluid dilatierbarer Ballon 7 ist am distalen Katheterende mit seinem proximalen Konus 8 am distalen Ende 5 des Außenschafts 4 und mit seinem distalen Konus 9 an einem Konuskörper 10 auf dem distalen Ende 11 des Innenschafts 6 druckdicht befestigt.

Das Ballonlumen 12 ist über den Ringraum 13 zwischen Außen- und Innenschaft 4, 6 mit einem Druckfluid beaufschlagbar.

In den Zeichnungen ist mit der der schraffierten Fläche zugeordneten Bezugsziffer 14 eine Beschichtung mit einem medizinischen Wirkstoff oder Stent angedeutet, die mithilfe des dilatierbaren Ballons 7 im Bereich der Stenose appliziert werden sollen.

Der Ballon-Katheter 1 ist schließlich an seinem distalen Ende mit einer Abdeckhülse 15 versehen, die mit ihrem proximalen Ende 16 auf dem Außenschaft 4 in längsaxialer Richtung verschiebbar geführt ist. Die Abdeckhülse 15 sitzt über dem in der Stellung gemäß Fig. 1 kontrahierten, zusammengefalteten Ballon 7 und schützt diesen zusammen mit der Beschichtung/dem Stent 14 beim Einführen des Katheters in das Körpergefäß 3. Wie aus Fig. 1 deutlich wird, tritt der Ballon 7 über die Frontöffnung 17 am distalen Ende der Abdeckhülse 15 aus, wobei zwischen der Frontöffnung 17 und dem Konuskörper 10 ein Initialöffnungsspalt 18 verbleibt, durch den der Ballonkonus nach außen treten kann.

Die Abdeckhülse 15 steht in proximaler Richtung über den Ballon 7 hinaus und bildet damit einen Zwischenraum 19 hinter dem Ballon 7, wo eine Durchlassöffnung 20 im Außenschaft 4 mit der durch den Ringraum 13 gebildeten Druckfluidleitung kommuniziert. Damit wird bei der Druckbeaufschlagung des Ballons 7 gleichzeitig der Zwischenraum 19 unter Druck gesetzt, sodass zwischen Ballon 7 und Abdeckhülse 15 ein Gegendruck erzeugt wird, der einem "Verkeilen" des Ballons 7 mit der Abdeckhülse 15 entgegenwirkt.

Mit Abstand A zum proximalen Ende 16 der Abdeckhülse 15 ist am Außenschaft 4 ein ringförmig umlaufender Anschlag 21 vorgesehen. Der Abstand A definiert dabei den maximal möglichen Verschiebeweg der Abdeckhülse 15 auf dem Katheter in proximaler Richtung.

Die Funktionsweise des Ballon-Katheters 1 ist nun wie folgt zu erläutern:
In der in Fig. 1 gezeigten Konfiguration wird der Ballon-Katheter bis zu der gezeigten Stenose 2 in dem Körpergefäß 3 vorgeschoben. Dabei ist die Beschichtung/der Stent 14 innerhalb der Abdeckhülse 15 sauber verwahrt, der Ballon 7 dabei auf seinem minimalen Durchmesser gehalten. Durch diese Verwahrung weist der Katheter ein geringflächiges Profil auch nach der Passage von engen Radien auf. Insbesondere tritt kein sogenanntes "Fishscaling" eines auf dem Ballon 7 sitzenden Stents 14 in engen Kurven auf. Ferner ist eine gesonderte Einbettung eines Stents 14 auf dem Ballon 7 aufgrund der Abdeckhülse 15 nicht mehr nötig.

Nach Erreichen der Soll-Position im Bereich einer Stenose 2, wie dies in Fig. 1 gezeigt ist, wird der Ballon 7 durch Einbringen eines Druckfluids über den Ringraum 13 mit Druck beaufschlagt. Der Ballon 7 beginnt, sich im Initialöffnungsspalt 18 auszudehnen, wodurch gleichzeitig eine Abstreifkraft F auf die Abdeckhülse 15 in axialer Richtung nach proximal erzeugt wird. Die Abdeckhülse 15 gleitet damit auf dem Außenschaft 4 zurück und gibt zunehmend unter weiterem Dilatieren des Ballons 7 diesen frei (siehe Fig. 2 und 3).

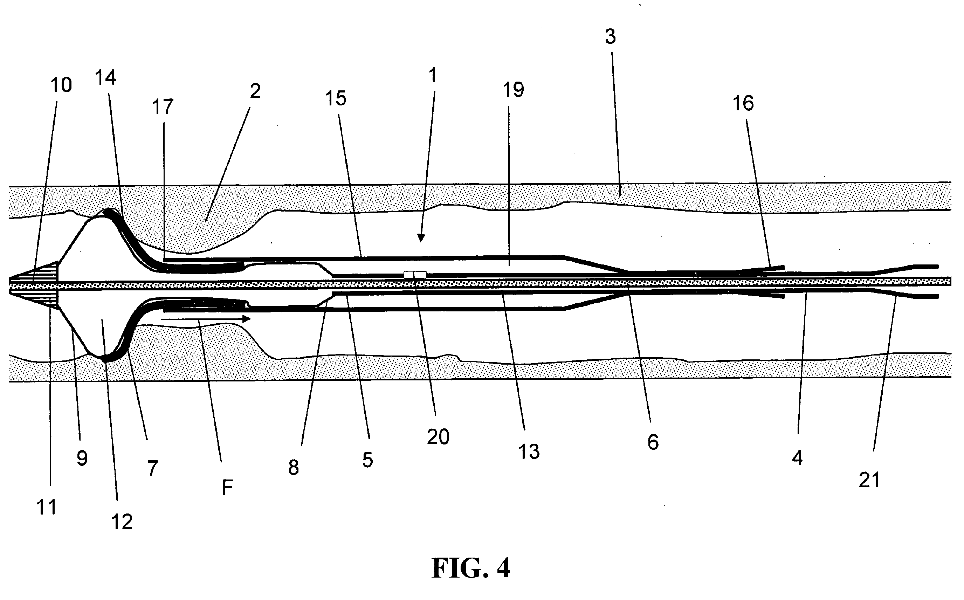

Da sich der Ballon 7 am distalen Ende des Katheters vor der Stenose 2 ausdehnt, wird der Ballon mit der Beschichtung/dem Stent 14 distal vor der Stenose 2 verankert (Fig. 4), sodass dann der Blutfluss durch das Körpergefäß 3 gestoppt ist. Wirkstoffe in der Beschichtung 14 werden damit nicht ausgeschwemmt, ihre Dosierung kann dort also geringer gehalten werden.

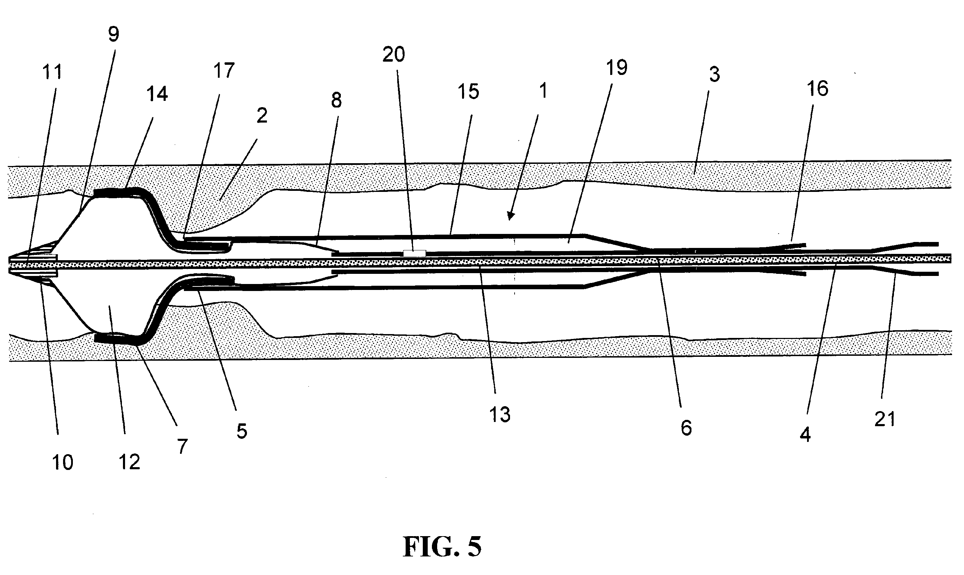

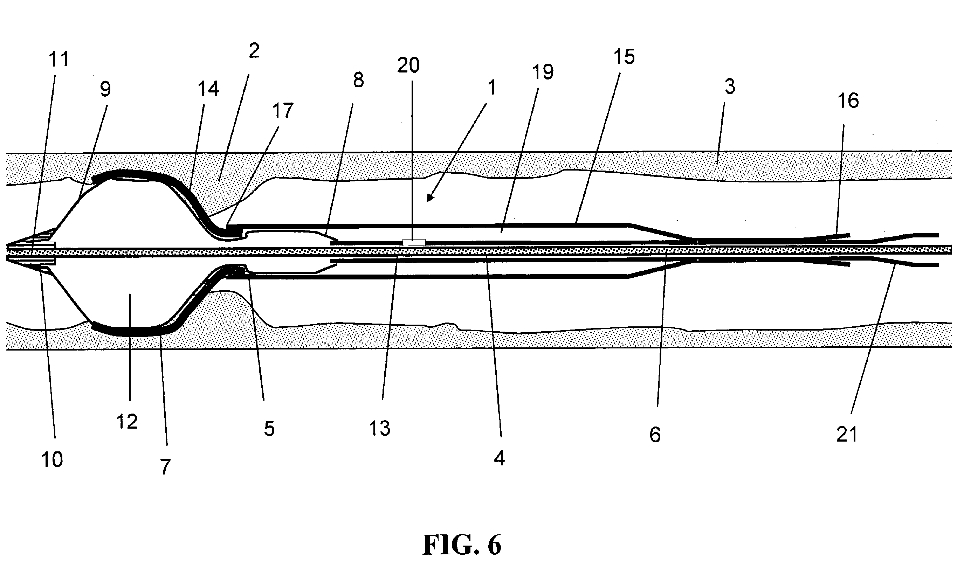

Mit zunehmender Aufweitung des Ballons 7 wird die Abdeckhülse 15 immer weiter nach proximal verschoben, die Beschichtung/der Stent 14 werden im Bereich der Stenose 2 ausgebracht und Letztere zunehmend aufgedehnt (Fig. 5 und 6).

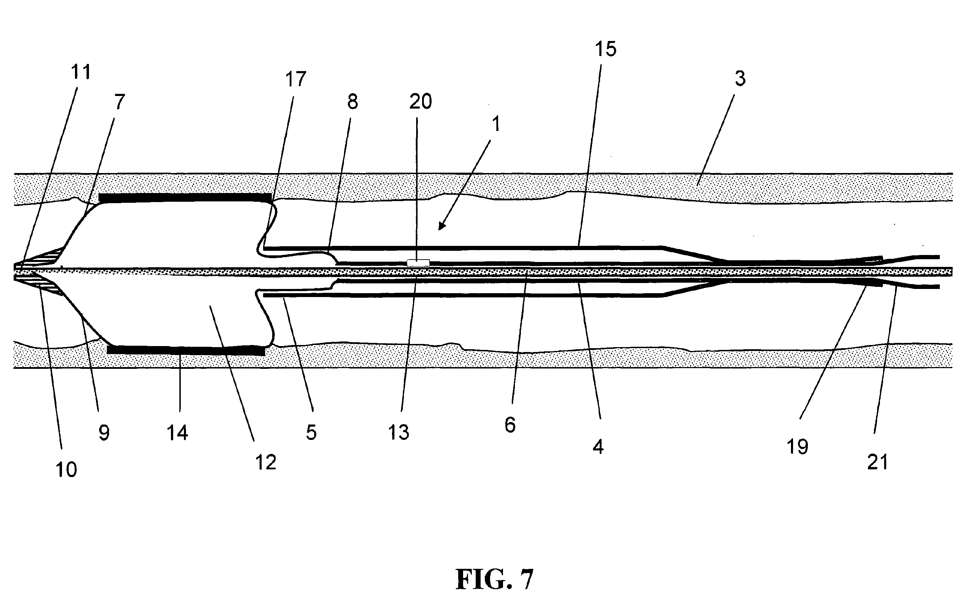

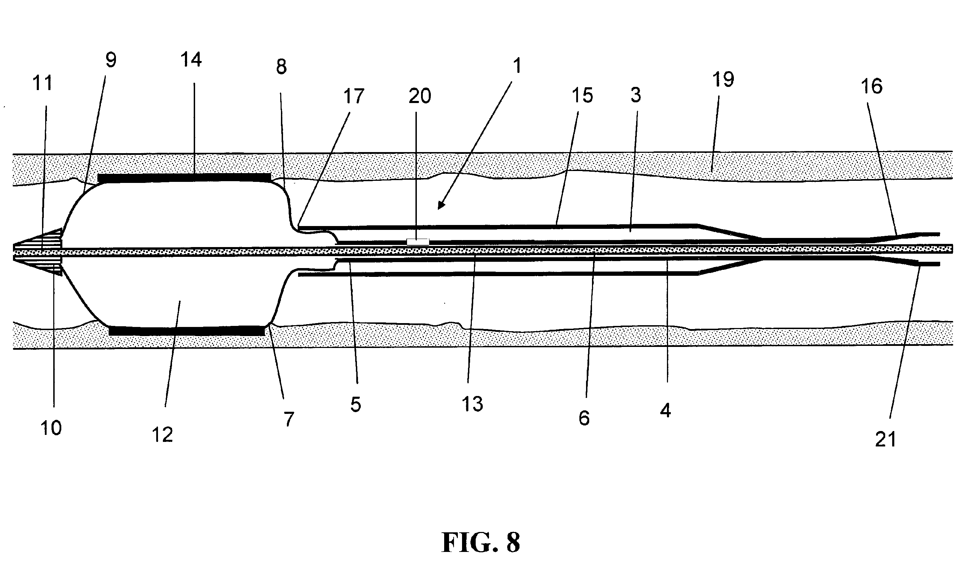

Im Endstadium der Dilatation des Ballons 7 schließlich ist die Beschichtung/der Stent 14 mithilfe des voll expandierten Ballons 7 am Körpergefäß 3 unter Behebung der Stenose 2 angelegt (Fig. 7), die Abdeckhülse 15 hat schließlich ihre Endposition (Fig. 8) am Anschlag 21 erreicht, wodurch sich der proximale Konus 8 des Ballons 7 an der Frontöffnung 7 abstützen kann. Der unter Druck stehende Zwischenraum 19 ist damit abgedichtet, sodass der Ballon 7 mit seinem vollen Solldruck beaufschlagt und komplett dilatiert werden kann.

Durch Druckentlastung kann der Ballon 7 sich wieder kontrahieren und der Katheter 1 aus dem Körper entfernt werden.

## Patentansprüche

1. Ballon-Katheter, insbesondere zum Ausbringen von Medikamenten, Stents oder medikamentenbeschichteten Stents im Bereich einer Stenose, umfassend
- einen Außenschaft (4),
- einen Innenschaft (6), und
- einen durch ein zuführbares Druckfluid dilatierbaren Ballon (7) am distalen Katheterende, der mit seinem proximalen Ende (8) am Außenschaft (4) und seinem distalen Ende (9) am über den Außenschaft (4) nach distal vorstehenden Innenschaft (6) angebracht ist,
**gekennzeichnet durch**
- eine in einer Abdeckstellung über dem kontrahierten Ballon (7) sitzende, auf dem Außenschaft (4) verschiebbar geführte Abdeckhülse (15), die **durch** den dilatierenden Ballon (7) unter dessen fortschreitender Freigabe von distal nach proximal über ihre Frontöffnung (17) auf dem Außenschaft (4) nach proximal verschiebbar ist,
- wobei der Ballon (7) über die Frontöffnung (17) am distalen Ende der Abdeckhülse (15) austritt.

2. Ballon-Katheter nach Anspruch 1, **dadurch gekennzeichnet, dass** die Abdeckhülse (15) auf dem Außenschaft (4) druckdicht abschließt.

3. Ballon-Katheter nach Anspruch 2, **dadurch gekennzeichnet, dass** der Zwischenraum (19) zwischen Abdeckhülse (15) und Ballon (7) mit dem zum Dilatieren des Ballons (7) eingesetzten Druckfluid ebenfalls unter Druck setzbar ist.

4. Ballon-Katheter nach einem der vorgenannten Ansprüche, **dadurch gekennzeichnet, dass** der Zwischenraum (19) zwischen Abdeckhülse (15) und Ballon (7) über eine Durchlassöffnung (20) im Außenschaft (4) mit der Druckfluidleitung zum Ballon (7) kommuniziert.

5. Ballon-Katheter nach einem der vorgenannten Ansprüche, **dadurch gekennzeichnet, dass** auf dem Ballon (7) ein Stent (14) angeordnet ist, der durch die Abdeckhülse (15) in ihrer Abdeckstellung in seinem kontrahierten Zustand gehalten und durch das Dilatieren des Ballons (7) bei gleichzeitigem Verschieben der Abdeckhülse (15) nach proximal selbsttätig oder durch den Dilatationsdruck freisetzbar ist.

6. Ballon-Katheter nach einem der Ansprüche 1 bis 5, **dadurch gekennzeichnet, dass** der Ballon (7) auf seiner Außenseite mit einer Medikamenten-Beschichtung (14) versehen ist, die durch das Dilatieren des Ballons (7) bei gleichzeitigem Verschieben der Abdeckhülse (15) nach proximal freisetzbar ist.

7. Ballon-Katheter nach einem der vorgenannten Ansprüche, **dadurch gekennzeichnet, dass** auf dem Außenschaft (4) ein Anschlag (21) zur Begrenzung des Verschiebeweges der Abdeckhülse (15) nach proximal angeordnet ist.

8. Ballon-Katheter nach einem der vorgenannten Ansprüche, **dadurch gekennzeichnet, dass** der Ballon (7) mit seinem distalen Ende (11) über einen Konuskörper (10) am Innenschaft (6) angebracht ist.

9. Ballon-Katheter nach Anspruch 8, **dadurch gekennzeichnet, dass** in der Abdeckstellung der Abdeckhülse (15) zwischen deren Frontöffnung (17) und dem Konuskörper (10) ein Initialöffnungsspalt (18) für den Ballon (7) verbleibt.

## Claims

1. A balloon catheter, in particular for delivering drugs, stents or drug-coated stents in the region of a stenosis, comprising:
- an outer shaft (4);
- an inner shaft (6); and
- a balloon (7) at the distal catheter end, which can be dilated by a pressure fluid that can be supplied and which at the proximal end (8) is attached to the outer shaft (4) and at the distal end (9) is attached to the inner shaft (6) protruding distally over the outer shaft (8),
**characterized by**
- a cover sheath (15), which is seated over the compressed balloon in a cover position and is guided displaceably on the outer shaft and which can be displaced by the dilating balloon (7), under the progressive release thereof from distal to proximal, via the front opening (17) of the sheath onto the outer shaft (4) in the proximal direction,
- wherein the balloon (7) exits at the distal end of the cover sheath (15) via the front opening (17).

2. The balloon catheter according to claim 1, **characterized in that** the cover sheath (15) is sealed on the outer shaft (4) in a pressure-tight manner.

3. The balloon catheter according to claim 2, **characterized in that** the intermediate space (19) between the cover sheath (15) and the balloon (7) can likewise be pressurized by the pressure fluid that is used to dilate the balloon (7).

4. A balloon catheter according to any one of the preceding claims, **characterized in that** the intermediate space (19) between the cover sheath (15) and the balloon (7) communicates with the pressure fluid line to the balloon (7) via a through-opening (20) in the outer shaft (4).

5. A balloon catheter according to any one of the preceding claims, **characterized in that** a stent (14) is arranged on the balloon (7), the stent being held in the compressed state thereof by the cover sheath (15) when the sheath is in the cover position and being releasable by dilation of the balloon (7), with simultaneous displacement of the cover sheath (15) toward proximal, either automatically or by the dilation pressure.

6. A balloon catheter according to any one of claims 1 to 5, **characterized in that** the outside of the balloon (7) is provided with a drug coating (14), which can be released by dilating the balloon (7) and simultaneous displacement of the cover sheath (15) in the proximal direction.

7. A balloon catheter according to any one of the preceding claims, **characterized in that** a stop (21) for delimiting the displacement path of the cover sheath (15) in the proximal direction is disposed on the outer shaft (4).

8. A balloon catheter according to any one of the preceding claims, **characterized in that** the distal end (11) of the balloon (7) is provided over a cone body (10) on the inner shaft (6).

9. The balloon catheter according to claim 8, **characterized in that** an initial opening gap (18) remains for the balloon (7) between the front opening (17) of the cover sheath and the cone body (10) in the cover position of the cover sheath (15).

## Revendications

1. Cathéter à ballonnet, en particulier pour la délivrance de médicaments, de stents ou de stents recouverts de médicaments dans la région d'une sténose, comprenant
- une tige extérieure (4),
- une tige intérieure (6), et
- un ballonnet (7) dilatable par un fluide de pression pouvant être alimenté, situé à l'extrémité distale du cathéter et fixé par son extrémité proximale (8) à la tige extérieure (4) et par son extrémité distale (9) à la tige intérieure (6) faisant saillie distalement au-delà de la tige extérieure (4),
**caractérisé en ce que**
- une gaine de recouvrement (15) guidée de façon coulissante sur la tige extérieure (4) et logée au-dessus du ballonnet contracté (7) dans une position de recouvrement, laquelle peut être déplacée de façon coulissante par son ouverture frontale (17) vers l'extrémité proximale sur la tige extérieure (4), par le ballonnet dilaté (7), sous la libération progressive de celui-ci de l'extrémité distale vers l'extrémité proximale,
- dans lequel le ballonnet (7) sort par l'ouverture frontale (17) à l'extrémité distale de la gaine de recouvrement (15).

2. Cathéter à ballonnet selon la revendication 1, **caractérisé en ce que** la gaine de recouvrement (15) se referme de façon étanche à la pression sur la tige extérieur (4).

3. Cathéter à ballonnet selon la revendication 2, **caractérisé en ce que** l'espace intermédiaire (19) entre la gaine de recouvrement (15) et le ballonnet (7) peut également être mis sous pression avec le fluide de pression utilisé pour dilater le ballonnet (7).

4. Cathéter à ballonnet selon l'une des revendications précédentes, **caractérisé en ce que** l'espace intermédiaire (19) entre la gaine de recouvrement (15) et le ballonnet (7) communique avec la conduite de fluide sous pression vers le ballonnet (7) par une ouverture de passage (20) dans la tige extérieure (4).

5. Cathéter à ballonnet selon l'une des revendications précédentes, **caractérisé en ce qu'**un stent (14) est monté sur le ballonnet (7), lequel est maintenu dans son état contracté par la gaine de recouvrement (15) dans sa position de recouvrement, et lequel peut être libéré par le déplacement simultané de la gaine de recouvrement (15) vers l'extrémité proximale ou par la pression de dilatation.

6. Cathéter à ballonnet selon l'une des revendications 1 à 5, **caractérisé en ce que** le ballonnet (7) est pourvu d'une couche contenant des médicaments (14) sur son côté extérieur, laquelle peut être libérée vers l'extrémité proximale par la dilatation du ballon (7) et le déplacement simultané de la gaine de recouvrement (15).

7. Cathéter à ballonnet selon l'une des revendications précédentes, **caractérisé en ce qu'**une butée (21) destinée à limiter le trajet de déplacement de la gaine de recouvrement (15) vers l'extrémité proximale est prévue sur la tige extérieure (4).

8. Cathéter à ballonnet selon l'une des revendications précédentes, **caractérisé en ce que** le ballonnet (7) est fixé par son extrémité distale (11) à la tige intérieure (6) par le biais d'un corps conique (10).

9. Cathéter à ballonnet selon la revendication 8, **caractérisé en ce que** dans la position de recouvrement de la gaine de recouvrement (15), une fente d'ouverture initiale (18) demeure pour le ballonnet (7) entre l'ouverture frontale (17) de celle-ci et le corps conique (10).
